# EUROPEAN PATENT APPLICATION

(11) **EP 3 524 977 A1**
(43) Date of publication of application: **14.08.2019**
(21) Application number: 17858007.2
(22) Date of filing: 25.05.2017
(51) Int. Cl.: G01N 33/48, G01N 1/10

(54) **PLASMA SEPARATION DEVICE AND PLASMA SEPARATION METHOD**

(30) Priority: 03.10.2016 JP 2016196059
(71) Applicant: CELLSPECT CO., LTD., Morioka-city, Iwate 0200857 (JP)
(72) Inventor: DOI Kazuhiko, Morioka-city Iwate 020-0857 (JP); KONDOU Shin-ichi, Morioka-city Iwate 020-0857 (JP); SUZUKI Hiroko, Morioka-city Iwate 020-0857 (JP); NAGANO Norihiro, Morioka-city Iwate 020-0857 (JP); HOJO Wataru, Morioka-city Iwate 020-0857 (JP)
(74) Representative: Office Freylinger
(86) International application number: PCT/JP2017/019507
(87) International publication number: WO 2018/066167

(57) **Abstract**

[Problem] To provide a plasma (or serum) separation apparatus and a plasma (or serum) separation method that enable quick, reliable, and low-cost separation of plasma from blood, even a minute amount of blood, at any place, without using a centrifuge separator, a pressurization/suction pump, or the like.

[Solution] A plasma separation apparatus comprises a blood separation part having a blood separation member, and a plasma collection part having a plasma collection member, wherein the blood separation member is mounted on a hydrophobic pedestal and includes a blood receiving area and a plasma separation area that is connected to the plasma collection part, and the plasma separation part has the cross-sectional area thereof gradually decreasing toward the plasma collection part. Here, "plasma" means "plasma or serum."

## Description

### Technical Field

The present invention relates to an apparatus and method for separating a liquid component from a bodily fluid or blood, especially for separating plasma (or serum) from blood. More particularly, the invention relates to a plasma (or serum) separation apparatus and a plasma (or serum) separation method which do not require a centrifuge, or a suctioning or pressurizing pump, or the like, yet are capable of simply and conveniently separating plasma (or serum) even from a minute amount of blood, at a low cost.

### Background Art

Medical diagnose through blood examination is routinely conducted because various clinical statuses of patient can be determined based on absence or presence and/or the concentration values of certain substances dissolved in the bodily fluid of patient, especially blood.

After collected from a patient, a blood sample, especially plasma or serum, can be analyzed to provide a lot of useful medical information in terms of, for example, chemical compositions and qualities, hematology, or coagulation. For example, a blood examination can be used to determine physiological and biochemical statuses of the patient in relation to a disease, such as mineral or certain DNA contents, drug potency, and organ functions. Such blood examination can be generally available in a clinical laboratory in a hospital, clinical examination company, and the like.

Note that, those samples to be examined, namely, plasma and serum, are similar to each other in their components, except that serum obtained from a coagulated blood specimen is free of fibrinogen and other certain coagulant factors lost as a result of coagulation process.

Conventionally, in order to analyze plasma or serum, objects of analysis (i.e., plasma or serum) must be separated from other blood components. For this end, it is usually required that a medical practitioner or the like collects about 10 mL of venous blood using a syringe, thereafter it is sent to an examination facility, where a centrifugal separation method is carried out for regulation and separation of a liquid component, such as plasma or serum, from blood cells or blood clots. This is a typical centrifugal separation method for separating blood into cellular components (e.g., red blood cells and white blood cells) and blood clots, and plasma or serum, by the difference of specific gravities. With this centrifugal separation method, in general, when no hemolysis has occurred and when an anticoagulant has been added, blood components are clearly separated in a centrifugal tube such that red blood cells having a heavy specific gravity would be separated to a lower layer (about 41% of whole blood), plasma having a light specific gravity would be separated to a upper layer (about 55% of whole blood), and platelets and white blood cells would be separated to a middle layer (about 4% of whole blood) . When no anticoagulant has been added, whole blood is separated into serum at the upper layer and blood clots at the lower layer through a centrifugal operation.

Because blood collection is a practice to be performed by a medical worker, it can impose a burden on both an objective person (i.e., the subject) and medical establishments, and can also increase costs. Furthermore, blood examination will require a centrifuge separator and operating staff therefor, taking an additional cost and labor.

Furthermore, a regulation of plasma or serum by the centrifugal separation often results in a problem of hemolysis. Occurrence of hemolysis is undesirable as it can cause enzymes, hemoglobin and other pigments, and stroma to be released into liquid components of blood. If such is the case, multiple clinical trials can be spoiled.

Thus, there have been needs for a plasma or serum separation apparatus and a plasma or serum separation method that enable analyzing substances contained in plasma or serum quickly, readily, and accurately, even if the amount of blood is very small and even if a user or a person concerned is not the medical worker with no training, without requiring a centrifugal separator method, a suctioning or pressurizing method, or the like.

There have also been needs for a simple, convenient, and low-cost plasma or serum separation apparatus and a plasma or serum separation method capable of dealing with global health care including developing regions and the like. (Hereinafter, "plasma or serum" may be simply referred to as "plasma.")

As for inventions with such objectives, blood separation apparatuses capable of separating blood without using a centrifuge separator have been proposed, for example in Patent Literature 1 and Patent Literature 2.

Patent Literature 1 discloses a configuration composed of a filter member for moving plasma more quickly than blood cells and a subsequent serially-connected plasma or serum separation film, or, a configuration composed of a first filter member for moving plasma more quickly than blood cells, a second filter member that is a plasma or serum separation film, and a third filter member for capturing fibrin and the like, in which blood components are separated by vacuum filtration, therefore it requires a system, apparatus, or manpower for depressurizing.

Patent Literature 2 discloses a configuration in which a liquid part of blood is separated from cellular components of blood as the blood flows through first and second matrixes, wherein the first, porous separation matrix contains binders for the cellular components of blood, and the second matrix is configured to allow for the liquid part of blood to flow into the first matrix by a capillary effect or chromatographic separation. Unfortunately, this type of plasma separator is difficult to be put into practical use because it takes time for separation, and cellular components such as red blood cells can be clogged to cause hemolysis and other problems.

Even if these conventional techniques do not require a centrifuge separator, they require an apparatus for depressurizing or pressurizing, or may need a combination of multiple filter members and porous matrixes, or can rise a risk of hemolysis, or may require another apparatus, tool, or manpower, or even if they do not need another apparatus, there is no guarantees that a liquid component of blood is collected quickly, simply, and conveniently in an amount just necessary for inspection at a low cost.

Furthermore, in medical diagnoses, it is requested that analysis is carried out simply and conveniently in a short time and at a lower cost. In particular, an important issue is to minimize the amount of specimen needed for analysis. If a system is established capable of preparing a specimen for biochemical examination by collecting a small amount of blood with a blood collection tool usable at home, the specimen could be swiftly sent to a blood examination facility and results could be received in a short time. Such system can deal with a growing worldwide need for total health care because it would be extremely useful not only for home health care but also in regions where medical establishments are inaccessible and even in developing counties. Presently, however, any such system that is practical and useful has not been developed yet.

As mentioned above, according to recent social needs, such the method and apparatus (instrument) has been needed for clinical care not only in medical institutions but also at home or in developing countries, etc. that can easily separate a blood component such as plasma or serum as a sample for blood examination from even a minute amount of blood, without requiring a centrifuge separator, a special apparatus or tool for suctioning or pressurizing, and manpower

Hereinafter, "plasma or serum" may be simply referred to as "plasma."

### Citation List

### Patent Literature

PTL 1: Japanese Patent Laid-Open No. 2004-344874
PTL 2: Japanese Patent Laid-Open No. 2006-177970

### Summary of Invention

### Problems to be solved by the invention

Considering the above-mentioned situations of the conventional arts, an object of the invention is to provide a plasma separation apparatus and a plasma separation method that enable quick, reliable, and low-cost separation of plasma from blood, even a minute amount of blood, at any place, without using a centrifuge separator, a pressurization/suction pump, or the like.

The above-mentioned object can be achieved by features defined in the claims.

Here, the term "plasma" means "plasma or serum."

A plasma separation apparatus according to the invention comprises, a blood separation part having a blood separation member, and a plasma collection part having a plasma collection member, wherein the blood separation member is mounted on a hydrophobic pedestal and includes a blood receiving area and a plasma separation area that is connected to the plasma collection part, and the plasma separation part has the cross-sectional area thereof gradually decreasing toward the plasma collection part.

The cross-sectional area of the plasma separation area in the plasma separation apparatus according to the invention is gradually decreased toward the plasma collection part by the end cutting.

The blood separation part of the plasma separation apparatus according to the invention comprises a blood reserving part on the upper surface side of the blood separation member for temporarily reserving blood.

The blood separation part and the plasma collection part of the plasma separation apparatus according to the invention constitute a core part, wherein the core part is contained in a housing part.

The plasma separation apparatus according to the invention further comprises a support member for separation, for separating into the blood separation part and the plasma collection part.

A blood examination kit according to the invention comprises the plasma separation apparatus and a blood collection tool.

A plasma separation method for separating plasma using a plasma separation apparatus comprising a blood separation part having a blood separation member and a plasma collection part having a plasma collection member, wherein the blood separation member arranged on a hydrophobic pedestal has a blood receiving area and a plasma separation area connected to the plasma collection part, a cross-sectional area of the plasma separation area is gradually decreased toward the plasma collection part, the plasma separation method comprising steps of separating the plasma from the blood received in the blood receiving area, in the plasma separation area, and collecting the separated plasma in the plasma collection part.

In the plasma separation method according to the invention, the cross-sectional area of the plasma separation part in the plasma separation apparatus is gradually decreased toward the plasma collection part by the end cutting.

In the plasma separation method according to the invention, the blood separation part of the plasma separation apparatus comprises a blood reserving part on the upper surface side of the blood separation member so that blood is received in the blood receiving area after temporarily being reserved in the blood reserving part.

The plasma separation method according to the invention comprises a step of separating the blood separation part and the plasma collection part after the plasma separated from the blood in the plasma separation area of the blood separation member of the plasma separation apparatus is collected in the plasma collection part.

### Advantageous Effects of Invention

The plasma or serum separation apparatus of the invention does not require a centrifuge separator, a suctioning or pressurizing apparatus, electric power, or the like, and can prepare a sample for blood examination easily, conveniently, quickly, and safely at a low cost, outside a hospital, at home, or in foreign countries where there is no medical facility around, and even by any ordinary person without technique training.

According to the apparatus, by a simple operation and without requiring a professional skill, a liquid component (plasma or serum) can be separated from even a minute amount of blood easily, quickly, and safely at a low cost without concern about hemolysis, even in a place where there is no sophisticated analytic instrument such as the centrifuge separator and the like, and even by a home user himself or herself. Not only the separated liquid component but also the cellular components can be used as a specimen for blood examination.

Here, the term "blood" is referred to as a synonym of whole blood and includes any combination of cellular components and liquid (non-cellular) components of blood. Typical cellular components include, but without limitation, red blood cells (erythrocytes), white blood cells (leukocytes), and platelets (thrombocytes), and any combination of these. White blood cells include mononuclear leukocytes, granulocytes, agranulocytes, and lymphocytes. When an anticoagulant has been added, typical liquid components include, but without limitation, plasma, dissolved salts and inorganics, and plasma proteins, and the like.

Those samples to be examined, namely, "plasma and serum" are similar to each other in their components, except that serum obtained from a coagulated blood specimen is free of fibrinogen and other certain coagulants lost as a result of coagulation process.

Moreover, in relation to blood separation, in general, a blood specimen is separated into a liquid component (plasma or serum) and cellular components (blood cells or blood clots) . Specifically, a blood specimen with an anticoagulant is separated into plasma and blood cells, while a blood specimen without an anticoagulant is separated into serum and blood clots.

Hereinafter, "plasma or serum" may be simply referred to as "plasma."

"Plasma collection rate" or "collection rate" is referred to as a value indicated by percentage of the amount of collected plasma relative to the total amount of blood dropped onto a blood separation part. When the separated liquid component is serum, then it is referred to as "serum collection rate."

### Brief Description of Drawings

Now preferable embodiments of the invention will be described with reference to the drawings.
Fig. 1 shows an external view of a core part of a plasma separation apparatus of an embodiment 1;
Fig. 2A shows an external view of the plasma separation apparatus of the embodiment 1; Fig. 2B shows a sectional view thereof;

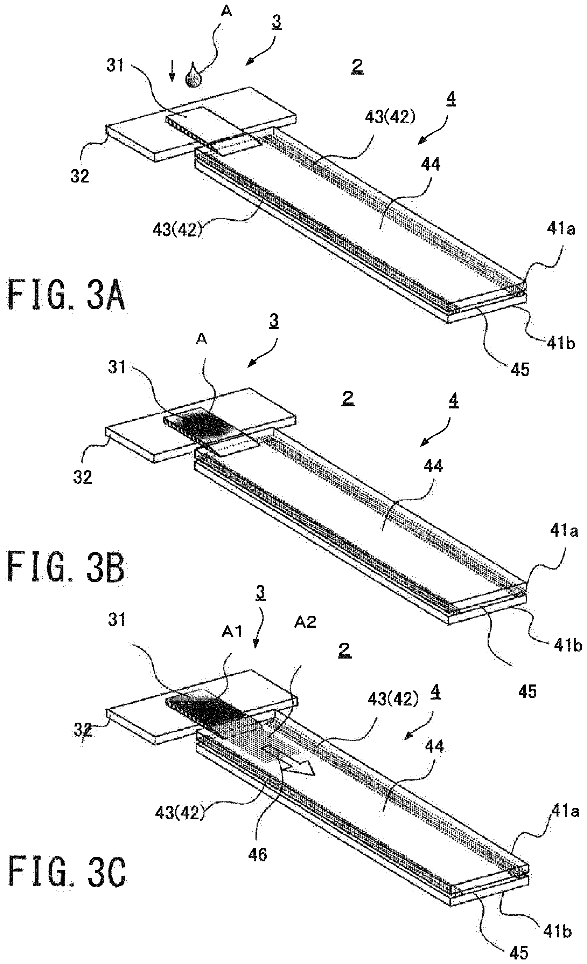

Figs. 3 is views roughly showing modes of forming of blood passage in the core part of the embodiment 1: Fig. 3A shows a mode of dropping blood onto a blood separation member; Fig. 3B shows a mode of infiltration of the dropped blood into the blood separation member, and Fig. 3C shows a mode of trapping blood cell constituent in the blood separation member and forming a collection part passage in a collection part by a liquid component in the blood through a communication/corporation between a passage forming action of the blood separation member and a capillary phenomenon of a gap of the collection part;
Figs. 4A to 4E show various modes of the blood separation member of embodiment 1, having different sizes and/or shapes of end;
Fig. 5 shows various modes of end working in a thickness direction of the blood separation member of embodiment 1;
Figs. 6 show modes of end width cutting of the blood separation member of embodiment 1; Fig. 6A shows a top view and Figs. 6B, 6C show sectional views respectively;
Fig. 7 shows an external view of a plasma separation apparatus of embodiment 2;
Fig. 8 shows a sectional view of the plasma separation apparatus of embodiment 2;
Fig. 9 shows an explosive view of the plasma separation apparatus of embodiment 2; and
Fig. 10 shows a perspective view of an external view of a core part (except for a support member for separation) after the core part of the plasma separation apparatus is assembled of embodiment 2.

### Description of Embodiments

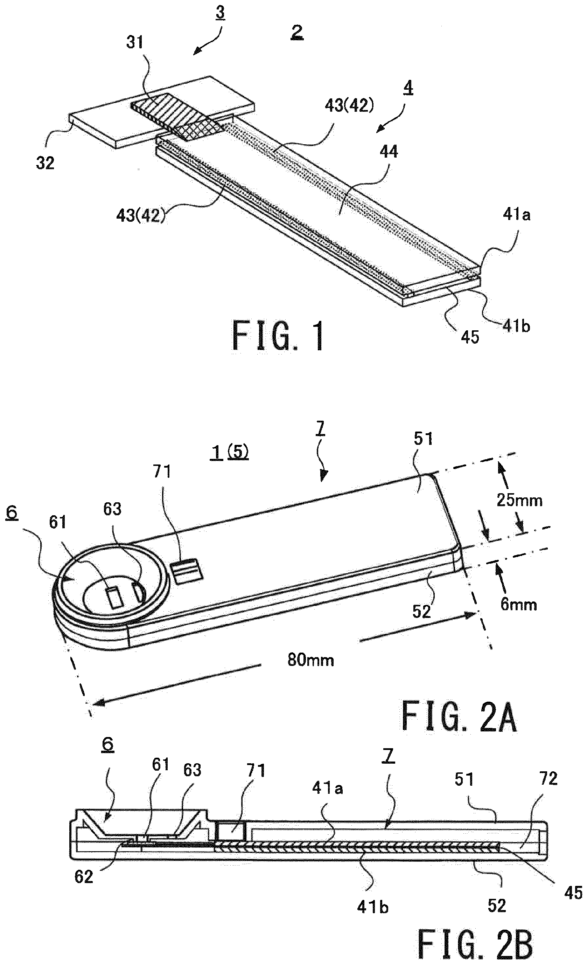

An apparatus according to the invention functions to separate components in liquid, as shown for example in Figs 1 to 10 in which its external view and the like are shown. Preferably the liquid is blood, and the components to be separated are roughly classified into cellular components (blood cells) such as red blood cell and white blood cell to be trapped in a blood separation member, and liquid components such as plasma or serum which contains substances (including DNA, RNA, and the like) to be applied to various biochemical examinations.

### (Embodiment 1)

One embodiment of the invention is a plasma or serum (hereinafter, simply referred to as "plasma") separation apparatus 1 comprising at least a core part 2 (Fig. 1) and an outer housing 5 (Fig. 2). The housing 5 includes a housing cover 51 and a housing base 52.

### <Structure of core part>

As seen in Fig. 1, the structure of the core part 2 of the plasma separation apparatus 1 of the invention generally comprises a blood separation part 3 and a plasma collection part 4.

At first, the blood separation part 3 includes at least a blood separation member 31 and a pedestal 32 for blood separation part for mounting (supporting) the blood separation member 31. In addition, the plasma collection part 4 having at least two plasma collection members 41 (a, b) includes adhesive member 43 sealing the edges 42 of the plasma collection members 41 in liquid-tight, and a plasma collecting area 44 formed being surrounded by the two plasma collection members 41 and the adhesive member 43.

When a blood sample is applied to the blood separation part 3, the blood is received in a blood receiving area on the upper surface of the blood separation member 31, then infiltrates into the blood separation member 31, so that a collection part passage 46 from a plasma separation area in the blood separation member 31 to the plasma collecting area 44 is formed (Fig. 3C).

Here, the blood receiving area and the plasma separation area are areas related to the blood separation member, the blood receiving area refers to an area on the upper surface of the blood separation member 31, on which blood is dropped for infiltrating into the member, the plasma separation area means an area of the blood separation member 31, where the infiltrated blood is separated into plasma and cellular components, this area is connected to the plasma collection part.

Materials for the blood separation member are not limited to any of embodiments in particular, for example, synthetic polymers or fibers made of glass having a small diameter of fiber, or layered products composed of porous polymeric sheets or thin nonwoven fabrics and the like. Any material can be used when they have function/effectiveness as the blood separation member.

Nevertheless, when the material adsorbs components to be measured in blood, it is desirable to apply a surface treatment to a material constituting a first filter member. As surface treatment agents, polyether-based or silicone-based lubricants, hydrophilic polymers such as polyvinyl alcohol or polyvinylpyrrolidone, or hydrophilic natural polymers, or polymeric surfactants, can be used, but the materials are not limited to those.

As the blood separation member, one to which a special binder such as antibody for capturing cellular components of blood is coupled, is included therein as well.

As materials for the pedestal for blood separation part, polyethylene, polystyrene, polycarbonate, polyethylene terephthalate, polypropylene, dimethylpolysiloxane, Teflon®, silicone, and ABS, and the like are considered, and it is preferable that those materials each have a hydrophobic surface as described below. Here, the term "hydrophobic" generally refers to a property of small affinity with water molecules.

As a plasma collection member, it is preferable to use a hydrophilic material such as glass or resin, or a material to which hydrophilic treatment has been applied, which has an effect to occur a capillary phenomenon in a gap between the collecting members (i.e., the plasma collecting area).

As adhesive member, an adhesive tape, an adhesive, and the like may be considered. Integral molding without using the adhesive may be carried out, when the adhesive member is made of the same material as that of the correcting member.

### <External and sectional view>

Fig. 2A shows an external view of the plasma separation apparatus 1 of the invention; Fig. 2B shows a sectional view thereof.

The housing part 5 containing the core part 1 is provided with at least a blood inlet part 6 and a collecting body part 7.

The blood inlet part 6 is provided on an upper part of the blood separation part 3 contained in the housing and is provided with a blood inlet 61, a blood reserving part 62, and a blood deployment observation window 63 and the like as needed.

The blood reserving part 62 has an effect of thereby forming a temporal blood reserving space (blood reservoir) . It is seated so as to prevent a part of blood after the blood being dropped from slipping over the upper surface of the blood separation member and leaking therefrom (while being unseparated without infiltrating into the blood separation member) and from flowing into the plasma collection part (from mixing of blood with the plasma, leaking of blood into the plasma).

In other words, a part of the dropped blood usually flows while forming a passage owing to a wicking phenomenon in the blood separation member, but the phenomenon is not a little occurred that the remaining blood floods over the surface of the separation member, and as a result, the part of the blood flows into the collection part without separation.

By providing the blood reserving part, slightly flooded blood on the surface of the member can be temporarily reserved as it is owing to surface tension at the blood reserving part, in this way a "temporal blood reservoir space" is formed, and thereafter the blood gradually flows into the separation member, thus a leaking phenomenon as mentioned above can be prevented.

Note that, the term "wicking (phenomenon or effect)" refers to a spontaneous transportation of blood that occurs in the fibrous or porous structure of the blood separation member, which transportation may be uni-, bi-, or omni-directional. This phenomenon allows a liquid to flow against gravity. Such effect is caused by an intermolecular attraction force between the liquid and a neighboring solid surface.

Although, in the present embodiment, the blood reserving part is placed in contact with the upper surface of the blood separation member 31, a close contact with the blood separation member 31 is not necessary but some gaps may be allowed to be formed.

The blood reserving part has a function as a guide member to form the temporal blood reservoir owing to surface tension, but some gap is allowed to be formed unless its effect as a temporal blood reservoir forming part (or guiding part for forming temporal blood reservoir) is lost, and unless blood slip over the upper surface of the blood separation member, leak therefrom, and flow into the collection part because of the gap.

Such blood reserving part can be of any shape, can be made of any material, and can be seated anywhere, when it maintains its effect as the above mentioned "temporal blood reservoir forming part (or guiding part for forming temporal blood reservoir)".

In embodiment 1, the blood reserving part 62 is formed in a convex part extending along a rectangle shape of the blood inlet 61 and perpendicularly extending downward therefrom, but it may be, for example, circular, comb-shaped, U-shaped, channel-shaped, or other shapes.

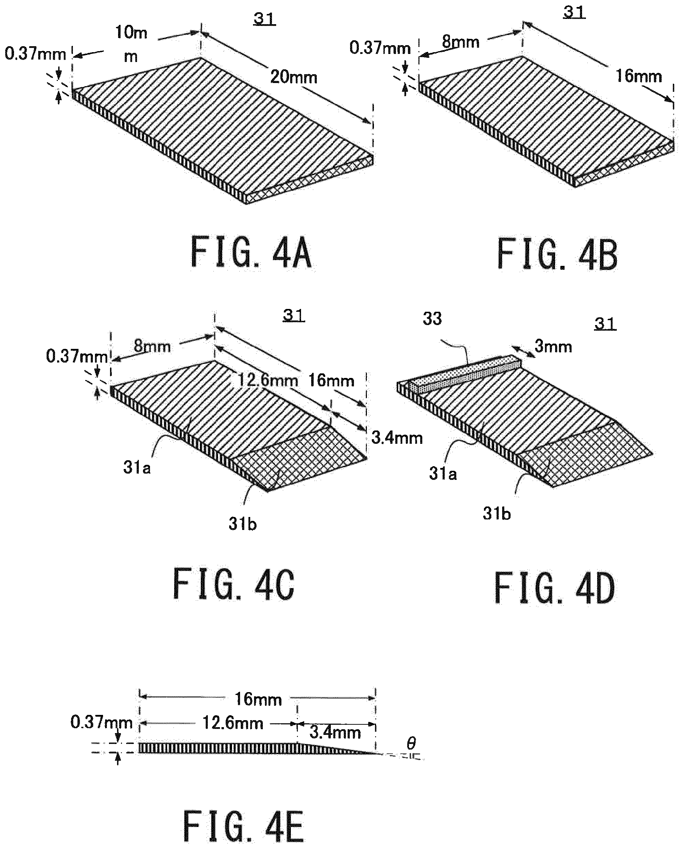

Alternatively, as another mode, it may be a bar-shaped member 33 shown in Fig. 4D. In such mode, the blood reserving part is preferably provided downstream of the blood dropping point (the plasma collection part side) to prevent blood from slipping over the upper surface of the blood separation member and leaking therefrom too.

As mention above, the blood reserving part may be provided on the lower surface of the blood inlet 61 integrally with the housing 5, like the blood reserving part 62, or it may be made as an independent structure separate from the blood inlet, when the effect as the blood reserving part is maintained. For example, the bar-shaped member 33 as shown in Fig. 4D may be provided with scaffolds or the like which is in contact with the pedestal 32 for blood separation part at the both ends and can be integrally molded with the pedestal 32 for separating part in a bridge shape.

The effect as a blood reserving part can be obtained not only by providing any member, but also by making its blood-contacting surface hydrophobic, for example. As an example, the effect of forming temporal blood reservoir can also be obtained by making hydrophobic the lower surface of the blood inlet 61 which may come in contact with dropped and flooded blood.

Note that the blood reserving part not only prevents blood from leaking over the upper surface of the blood separation member, but also has a function to more effectively exhibit the blood separating function of the blood separation member.

Whereas the collecting body part 7 is provided with a plasma deployment observation window 71, an opening 72 or the like. as needed (see Fig. 2). The opening 72 may be provided with a film that allows air to pass therethrough but not liquid, a valve, a connector to an examination system or the like, or a liquid (plasma) reserving part. Alternatively, the opening 72 is not necessarily required such that it is a sealed configuration.

<Method for separating plasma from blood without requiring centrifuge separator, suctioning or pressurizing apparatus, or the like> See Fig. 3.

A method for separating plasma from blood using a plasma separation apparatus of the invention comprises at least the following steps:
1. A step for providing a plasma separation apparatus, wherein the end of the blood separation member is inserted into (arranged in) a gap of the plasma collection part, the blood separation member being included in the blood separation part and mounted on a hydrophobic pedestal, and a cross-sectional area of the end being gradually decreased toward a plasma collection part (preparation of a blood separation apparatus) ;
2. A step for introducing blood to the blood separation member through a blood reserving part serving as a temporal blood reservoir (a step for applying blood);
3. A step for separating blood introduced in the blood separation member, wherein the blood is separated into blood cellular components and plasma while forming a passage in the blood separation member owing to a wicking effect in the blood separation member (a step for blood deployment and separation) ;
4. A step for collecting plasma in the plasma collection part, wherein the separated plasma forms a passage in the gap of the plasma collection part through the communication/corporation between the wicking effect in the blood separation member and a capillary phenomenon in the plasma collection part, so that the plasma is collected in the plasma collection part (a step for collecting plasma).
   The following step is the last for conducting a final examination.
5. A step for sending the plasma collection part to the examination facilities for examination.

Because this method does not require any special apparatus such as a centrifuge separator, a suctioning or pressurizing apparatus (tool), or the like and can be performed by an ordinary parson without training who is not a medical worker, it can deal with needs of a person at home, a resident in a remote place or in developing countries, or a minority or a majority of people, plasma separation can be performed easily and quickly and separated plasma can be send to the examination facilities as it is. Thus, the apparatus is very useful.

In addition, as mentioned above, the term "wicking (effect)" refers to a spontaneous transportation of liquid that occurs in the fibrous or porous structure of the blood separation member, which transportation may be uni-, bi-, or omni-directional. This phenomenon allows the liquid to flow against gravity. Such effect is caused by an intermolecular attraction force between the liquid and a neighboring solid surface.

### <Mechanism for separation of blood >

Blood is dropped through the blood inlet 61 of the blood inlet part 6 and received in the blood receiving area on the upper surface of the blood separation member 31 that is arranged perpendicular to a blood filling direction. Since the blood does not infiltrate into the blood separation member immediately after dropping, an overflown part of the blood onto the upper surface of the blood separation member (i.e., the blood receiving part) is temporarily received in the blood reserving part 62 functioning as a temporal blood reservoir forming part (or a guiding part for forming temporal blood reservoir). Meanwhile, the blood gradually infiltrates into and spreads through the blood separation member 31 owing to the wicking effect caused by the various matrix structure of the blood separation part in the blood separation member 31, as a result, a force to push the blood toward the plasma collection part 4 connected with the end of the blood separation member 31 is generated, so that a collection part passage 46 is formed from the blood separation part 3 to the plasma collecting area 44 through the communication/corporation of the force with the capillary phenomenon of the gap of the plasm collection part 4.

Cellular components in blood (such as red blood cells and white blood cells) are trapped in the blood separation member 31. A liquid component of the blood (such as plasma or serum) flows into the plasma collecting area 44 of the plasma collection part 4 through the collection part passage 46 formed through the communication/corporation between the wicking effect of the blood separation part in the blood separation member 31 and the capillary phenomenon of the gap of the plasma collection part, thereby separated from the cellular components and collected.

Note that, although the reference signs are affixed to the drawings in order to help understanding the description, the present mechanism is not limited by the reference signs or not limited by any of embodiments.

### <Experiment 1> (Surface properties of the pedestal)

Object: To prevent blood applied to a blood separation member from adhering to the pedestal and resulting in a dead volume.

Method: In the pedestal made of silicon, after a hydrophobic coating is applied to at least an upper surface in contact with a planar blood separation member as shown in Fig. 1 by applying a hydrophobic coating agent or a hydrophobic film, or the like, an angle of contact of a water droplet on the pedestal is confirmed to be large enough to show that the pedestal is hydrophobic, thereafter blood is applied to the blood separation member, and a degree of adhesion of blood (blood cells and blood clots), etc. to the pedestal is confirmed.

Not that, coating is performed by applying a fluorine-based hydrophobic coating agent (available from NIPPECO Co. Ltd.) to the surface of the pedestal and dying to give hydrophobicity thereto, this time.

Results: Blood adherence (blood cells and blood clots) to the pedestal was less than a comparative example and blood components was more smoothly separated, and collection amount of the plasma as such was improved. With application of the hydrophobic coating to the surface of the pedestal, blood was prevented from adhering thereto and its dead volume was thereby decreased.

Note that, for hydrophobic coating, a commonly used material and method can be adapted when the pedestal is thereby made hydrophobic and the problem is not occurred that the coating itself is dissolved out by blood.

The material of the pedestal does not need to be silicon, and a hydrophobic material, or other materials to which hydrophobic coating is applicable and by which the pedestal is formed, will do.

In each of experiments of the embodiments, including comparative examples, a plasma collection time is generally 3 to 5 minutes, preferably 3 minutes, after blood is dropped.

If plasma collection time is too short, the plasma collection amount will be too small. If it is too long, an inflow of blood is likely to occur. A desirable plasma collection time can be set based on a constitution of each embodiment, size of each part, material of each member, and the like.

### <Experiment 2> (Volume of blood separation part)

Object: To consider the volume of a blood separation member in order to separate 100 µL of blood quickly and to collect only liquid component in the collection part.

Method: A plasma collection rate is calculated by using MF1 (material: bound glass fiber) available from GE Healthcare Japan Co., Ltd. as a blood separation member and a relationship between the plasma collection rate (hereinafter also referred to as "collection rate" or "yield") and the volume of the member is examined.

Note that, in embodiments of the invention, MF1 (product name) available from GE Healthcare Japan Co., Ltd. is used as one example of blood separation member, unless otherwise mentioned.

Results: when the blood separation member had a volume of 74 mm³ (20 mm (length) × 10 mm (width) × 0.37 mm (height (thickness)): Fig. 4(a)), plasma could be collected from 150 µL of blood; but plasma was hardly collected from 100 µL of blood.

Since a liquid holding capacity of the blood separation member is tending to depend on the volume. Based on the above result in case of the volume of 74 mm³, and considering an easily working shape of the end to be worked in the following experiment 3, the volume of the blood separation member for 100 µL of blood is 16 mm (length) × 8 mm (width) × 0.37 mm (height) (see Fig. 4B), it is about 47.36 mm³ in volume when converted.

### <Experiment 3> (Shape of the collection part of the blood separation member in contact with the collection part: the end cutting in thickness (direction))

Object: Since the thickness of the blood separation member used in the experiment 2 is greater than the height of gap of the collection part, the end of the blood separation member cannot be inserted into the gap of the collection part, and the plasma is hard to collect, therefore, the object of the experiment is to consider "cutting angle of the end in the thickness direction" (hereinafter, also referred to as "end thickness cutting angle") required for a quick separation of 100 µL of blood and collection of plasma, by decreasing the "thickness" of the end of the blood separation member, in other word, by decreasing the "thickness" of the end of the blood separation member through the end cutting at various acute angles (angles tapering the end in a shape of a triangle),
Note that the above-mentioned cutting in the thickness direction at an angle tapering in the shape of a triangle is hereinafter also referred to as "(end thickness cutting in a triangle" or simply "(end thickness cutting)."

Method: Since the thickness of the blood separation member used in experiment 2 is greater than the height of the gap (space) between the two collecting members of the plasma collection part (here, 0.15 mm, hereinafter also referred to as a "gap of the collection part"), in order to improve a contact between the gap of the collection part and the end of the blood separation member, the end of the blood separation member is cut at different acute angles, in other word, the end is subject to the end thickness cutting, so that the "thickness" of the end of the blood separation member is decreased.

And with that, the plasma collection rates of the blood separation member subjected to the end thickness cutting is compared with one without being subjected thereto.

**[Table 1]**

| End thickness cutting angle | 5° | 6.7° | 7° | 10° | 20° | 60° | 80° | 90° |
|---|---|---|---|---|---|---|---|---|
| Plasma collection rate | 12% | 30% | 30% | 12% | 6% | 0% | 0% | 0% |

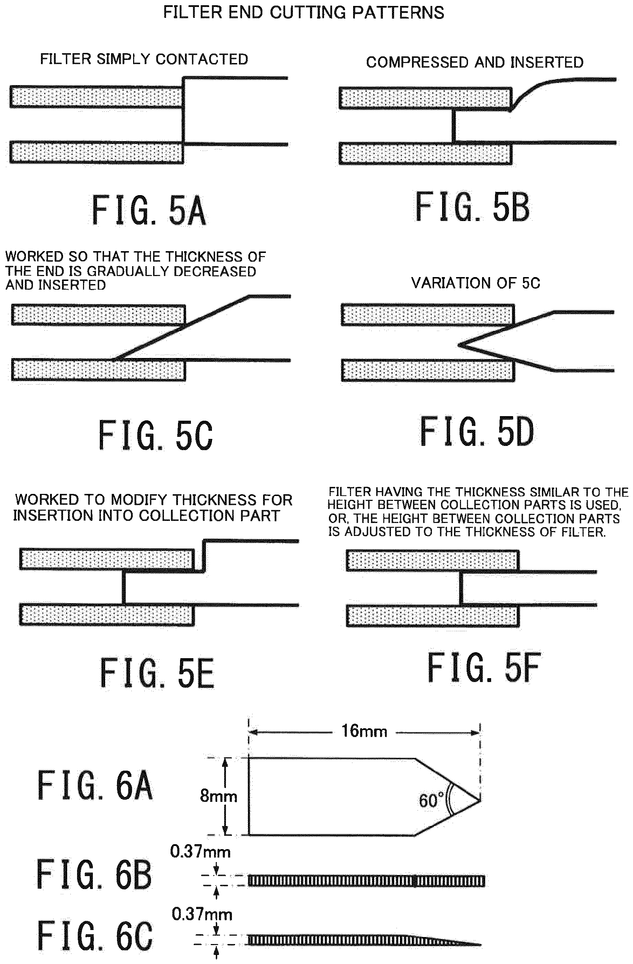

Results: See Table 1.
(1) Comparative example (without end cutting): when the end was not worked in any way (see Fig. 5A), since the thickness of the end (0.4 mm) was greater than the gap of the collection part (0.15 mm), and end of the collection part could not be inserted into the gap of the collection part, a blood separation experiment was conducted while the end was in a close contact with the inlet end of the collection part. However, as a result, no plasma was collected (see Table 1, the result for 90°) . Unless at least the end of the blood separation member was inserted into the gap, the plasm could not be collected due to a contact failure caused.
(2) Comparative example (end compressing) : To address the issue above, in an attempt to insert at least the end of the blood separation member into the gap of the collection part, a slight pressure (in the perpendicular direction) was applied to the end of the blood separation member to compress it, so that the end was inserted into the gap of the collection part (see Fig. 5B), and this case was considered.
   According to an investigation regarding this case, however, blood was not separated and plasma was not collected (almost zero).
(3) Experimental example (end thickness cutting) : As one mode of work for inserting the end of the blood separation member into the gap of the collection part without compressing the end of the blood separation member, the end thickness cutting was carried out at different acute angles by a file, so that the "thickness" of a part in a length of 3.4 mm of the end of the blood separation member was decreased as approaching a tip end. (see Fig. 4C).

The angles totaled in seven: 5°, 6.7°, 7°, 10°, 20°, 60°, 80°, and 90° (without cutting).

As indicated in Table 1, when 100 µL of blood was applied, the plasma collection rates were: 12% at 5°, 30% at 6.7°, 30% at 7°, 12% at 10°, 6% at 20°, 0% at 60°, 80°, and 90° of end thickness cutting angles (see Table 1).

Surprisingly, the plasma collection rates were particularly excellent when the end thickness cutting angles of the blood separation member were 6.7°-7°.

From the above results, it was proved that, in order to collect plasma from 100 µL of blood using the blood separation apparatus of the invention, the end thickness cutting angle was preferably not less than 5° and not more than 20°, more preferably not less than 5° and not more than 10°, even more preferably not less than 6.7° and not more than 7°.

In summary, when plasma is collected from 100 µL of blood, an optimal plasma collection rate can be obtained by the blood separation member having the dimensions of the blood separation member of 16 mm (length) × 8 mm (width) × 0.37 mm (height (thickness)), and by applying the work to the end at "the end cutting angle" of not less than 6.7° and not more than 7°, so that the thickness of the end is gradually decreased toward the tip end

Here, as for the shape of end obtained by the end thickness cutting for the blood separation member, various variations as shown in Figs. 5 are thinkable, but a result is given that patterns A and B produced a low or almost zero plasma collection rate, as mentioned above.

Note that, in embodiments of the present invention, experiments were carried out on an assumption that the blood separation member having dimensions of 16 mm (length) × 8 mm (width) × 0.37 mm (thickness) and with the end cut at the thickness cutting angle of 7° (hereinafter, may be abbreviated as "the thickness of 0.37 mm, the end thickness cutting angle of 7°") was used, unless otherwise mentioned (see Fig. 4C).

### <Experiment 4> (Prevention of blood from slipping over surface of blood separation member)

Object: As mentioned above, when 100 µL of blood was directly dropped onto the blood separation member, because a phenomenon was observed that a overflown part of blood slipped over the upper surface of the blood separation member without infiltrating into the blood separation member (blood leaking phenomenon) and directly flowed into the plasma collection part as it was, so that the collected plasma and cellular components such as red blood cells were intermingled.
Accordingly, the object is to consider means for preventing the slipping of blood on the surface of the blood separation member.

Method: The blood reserving member (made of hydrophobically treated glass, this time) having a shape as shown in Fig. 4D for example, is placed on the upper surface of a blood separation member, the blood is dropped on an upstream side of the blood reserving member (opposite side to the plasma collection part), and results of the plasma collection is compared.

Results: When the blood reserving member was placed as mentioned above and blood was dropped, whole blood did not infiltrate into the blood separation member (whose thickness was 0.37 mm and the end thickness cutting angle is 7°), but a part of it overflowed on the surface of the blood separation member, nevertheless, such blood was reserved temporarily in the blood reserving member by surface tension (the blood reserving member functioned as a temporary reservoir), so the blood did not leak nor slide into the plasma collection part from the surface of the blood separation member, whole blood eventually infiltrated into the blood separation member, so that the blood components were separated.

When the blood reserving member was absent, blood inflow (leakage) from the surface of the blood separation member to the plasma collection part was observed, in contrast, when the blood reserving member was present, blood inflow to the plasma collection part did not observed, the plasma collection rate was 30% which showed a good result (see Table 2).

Accordingly, from the above, it is understood that it is preferable to provide a member which can serve as a temporal blood reservoir forming part (guiding part for forming temporal blood reservoir) to prevent the above-mentioned blood leaking phenomenon on the surface of the blood separation member.

**[Table 2]**

| Reservoir | presence | absence |
|---|---|---|
| Plasma collection rate | 30% | * |

| | | |
|---|---|---|
| *: Blood inflow occurred. | | |

In embodiment 1, there is provided the blood reserving part 62 which is a rectangular protrusion at a lower part of the blood inlet 61 of the blood inlet part 6 in order to prevent the above-mentioned leaking phenomenon over the surface of the blood separation member. The blood reserving part may be any other shape such as a cylindrical shape, when it is effective as a temporal blood reservoir forming part (guiding part for forming temporal blood reservoir) as mentioned above.

Note that a blood reserving part was provided in other experiments as well (Experiments 1 - 3, 5 - 13).

### <Experiment 5> (Consideration of thickness of blood separation member)

Object: To consider differences in collection rate for blood separation members of the same material but having different thicknesses.

Method: With respect to the blood separation member, differences in collection rates of the plasma from 100 µL of blood is examined, when its thicknesses vary while its vertical length of 16 mm and the end thickness cutting angle of 7° is fixed.

In particularly, since it was understood from the previous experiment that the volume was an important factor, three types of blood separation members having the thicknesses of 0.25 mm, 0.37 mm, and 0.78 mm, respectively, are used (available from GE Healthcare Japan Co.Ltd., Product names: LF1, MF1, and VF2, respectively), and the widths are set to 12 mm, 8 mm, or 3.7 mm, so that a volume :16 mm (length) × width × thickness was approximately 47 mm³, which was the same condition as the case where 16 mm (length) × 8 mm (width), and the end thickness cutting angle of 7°are adapted except the width dimension. In other words, the collection rates are compared by dropping 100 µL of blood on the blood separation members each having a thickness of 0.25 mm, 0.37 mm, or 0.78 mm and length × width × thickness (in short, the volume) of approximately 47 mm³.

Results: As indicated in Table 3, when 100 µL of whole blood was dropped, a high plasma collection rate of 30% was obtained in case of the thickness of 0.37 mm, but plasma was not collected in other than the case, that is, the thicknesses of 0.25 mm or 0.78 mm. Moreover, it was understood that the collection rate significantly declined when the amount of the dropped blood was not near 100 µL.

As above, it was understood that the thickness of 0.37 mm was preferable when the amount of blood was 100 µL.

Here, as mentioned above, since the volume of the blood separation member is related to the collection rate, to collect plasma from 100 µL of blood, it is proven that, considering in terms of volume, the volume of 32 mm³ (where the thickness is 0.25 mm; 16 mm (length) × 8 mm (width) × 0.25 mm (thickness)) or less is small, the volume of 99.84 mm³ (when the thickness was 0.78 mm; 16 mm (length) × 8 mm (width) × 0.78 mm (thickness)) or more was too large; but the volume of 47.36 mm³ (when the thickness was 0.37 mm; 16 mm (length) × 8 mm (width) × 0.37 mm (thickness)) is the most effective, a large effect being 30%of the collection rate can be obtained.

Note that, here, for the ease of understanding, the volume of the blood separation member was not the volume after applying the end thickness cutting at angle 7°, but the volume before applying the end thickness cutting at angle 7°. The same is true for the following experiments too. (Incidentally, the volume after cutting at 7° is 42.9 mm³.)

**[Table 3]**

| Thickness of blood separation member | 0.25 mm | 0.37 mm | 0.78 mm |
|---|---|---|---|
| Plasma collection rate | 0 | 30% | 0 |

### <Experiment 6> (Relation between volume of blood separation member and amount of dropped blood in plasma collection rate - 1)

Object: To consider the relation between the volume of blood separation member and the amount of dropped blood - 1

Method: the plasma collection rates of the blood collection member to which the thickness cutting at 7°are applied, are compared, when amount of dropping blood are varied as 50 µL, 70 µL, 100 µL, 125 µL, while the volume (16 mm × 8 mm × 0.37 mm = 47.36 mm³) is fixed.

Results: When length × width × height (thickness), namely volume of the blood separation member was fixed, an optimal plasma collection rate of 30% was obtained when 100 µL of blood was dropped, but the plasma collection rate was 6.9% when 75 µL of blood was dropped, and the plasma was not collected and any effect could not be obtained at all when 50 µL or 125 µL of blood was dropped (see Table 4).

Accordingly, in the blood separation member to which the end thickness cutting at 7°was applied, when its volume was set to be 47.36 mm³ , it is important and preferable to set the blood dropping amount to 75 µL - 100 µL, and more preferably to set to 100 µL. In other words, when the blood dropping amount is 100 µL, it is important that the volume of the blood separation member is preferably set to near at 47.36 mm³.

Note that, for the ease of understanding, the volume of the blood separation member referred to here was one before applying the end thickness cutting at 7°. The same is true for the following experiments too.

**[Table 4]**

| Amount of whole blood | 50 µL | 75 µL | 100 µL | 125 µL |
|---|---|---|---|---|
| Plasma collection amount (µL) | 0 | 5.2 | 30 | 0 |
| Plasma collection rate (%) | 0 | 6.9 | 30 | 0 |

### <Experiment 7> (Relation between volume of blood separation member and amount of blood - 2)

Object: To consider an optimal volume of a blood separation member for 150 µL of whole blood, corresponding to the optimal volume of blood separation member (16 mm × 8 mm × 0.37 mm = 47.36 mm³) for 100 µL of whole blood.

Method: Examination is carried out as varying sizes of length × width, while the thickness is fixed at 0.37 mm. (End thickness cutting at 7°is adapted as well.)

Results: As a final result, as indicated in Table 5, "16 mm × 8 mm × 0.37 mm = 47.36 mm³" is preferable for 100 µL of whole blood, whereas "20 mm × 10 mm × 0.37 mm = 74 mm³" is preferable for 150µL of whole blood.

In other word, when whole blood is 150µL, the volume of the blood separation member is preferably 74 mm³ - 99.84 mm³, and more preferably, 74 mm³.

Note that as mentioned above, for the ease of understanding, the volume referred to here of the blood separation member was one before applying the thickness end cutting at 7° thereto.

**[Table 5]**

| Area | Amount of whole blood | Correction rate | Volume |
|---|---|---|---|
| 20 × 10 mm² | 150 µL | > 20% | 74 mm³ (20 × 10 × 0.37) |
| 16 × 8 mm² | 100 µL | 30% | 47.36 mm³ (16 × 8 × 0.37) |
| 16 × 8 mm² | 150 µL | 14.8% | 99.84 mm³ (16 × 8 × 0.78) |

From the results of Experiments 6 and 7, it is understood that the relation between the amount of applied blood and the volume of a blood separation member is very important with respect to plasma collection rates.

### <Experiment 8> (Consideration of material for plasma collection member)

Object: To consider the differences in plasma collection rates depending on the materials for the plasma collection member.

Method: In the previous experiment, glass was used for the material of the collecting member, but here the plasma collection rates are compared when PC, PET, PS, PVC, and PP are used as alternative material.

As the regular blood separation member, one having the thickness of 0.37 mm and with the end thickness cutting angle of 7° is used, and conditions of members other than the collecting member are the same.

Results: At first, when the collecting member was made of glass, good plasma collection rate of 30% could be always obtained (see Table 6).

In contrast, when the collecting member was made of material other than glass, its result was that the plasma collection rates were 10% for PS (polystyrene), 6.3% for PET (polyethylene terephthalate), 4.5% for PC (polycarbonate), 0.9% for PP (polypropylene), and 0% for PVC (polyvinyl chloride) (see Table 6).

With that, since the surface of the glass is hydrophilic, a similar experiment was performed after applying a hydrophilic treatment to the surface of the plasma collection member made of PC. After applying the hydrophilic treatment, the plasma collection rate 20% or more, which was a high value near that of the glass surface, was obtained, even though the plasma collection rate was only 4.5% before applying the hydrophilic treatment.

As mentioned above, it is understood that a high plasma collection rate is obtained, when the plasma collection member is made of hydrophilic glass or a PC material to which the hydrophilic surface treatment is applied. Therefore, it is preferable to use hydrophilic materials such as glass or resin, or material to which hydrophilic treatment is applied, and capable of causing capillary phenomenon in a gap (plasma collecting area) between the collecting members.

**[Table 6]**

| Collection part material | Glass | PC | Hydrophilized PC | PET | PS | PVC | PP |
|---|---|---|---|---|---|---|---|
| Plasma collection rate | 30% | 4.5% | 20% or more | 6.3% | 10% | 0% | 0.9% |

### <Experiment 9> (Consideration of presence/absence of opening in plasma collection part and end thickness cutting angle of blood separation member)

Object: To consider an influence of presence/absence of an opening (vent) in the plasma collection part due to differences in end thickness cutting angles of the blood separation member on the plasma collection rate.

Method: The plasma collection rates from 100 µL of blood is examined
by combining presence/absence of an opening (vent) in the plasma collection part and blood separation members having various end thickness cutting angles (angles tapering toward the tip end in a shape of a triangle).

Here, an absence of a vent (opening 45) means a mode in which the opening 45 is sealed with some means such as a film or the like. For example, the opening 45 can be sealed by a same adhesive as an adhesive 43 for the plasma collection members or sealed by the same material as the collecting members. However, it is needed to secure an air passage. In the experiment, the opening 45 is sealed with the adhesive, but the width of the plasma collecting area 44 is made greater than that of the blood separation member and partially made hydrophobic in order that an air passage is secured.

Results:
(1) The plasma collection rate was 30% when the "blood separation member having the thickness of 0.37 mm and the end thickness cutting angle of 7°" in Experiment 2 was used, and when the vent was present in the collection part. In contrast, when the vent was absence in the collection part, the plasma collection rate was lower to 13.5% but was not 0%, from this, it was understood that plasma could be satisfactorily collected to some extent (Table 7).
(2) Further, from a result of an examination regarding to a relation of presence/absence of the opening (vent) in the plasma collection part with the blood separation member, which was performed at different end thickness cutting angles, it was understood that, as indicated in Table 8, when the end thickness cutting angle of the blood separation member was preferably between 5° and 20° (i.e., 5°, 7°, 10°, and 20°), then even though the vent was absent, plasma could be collected to some extent. It was found that, more preferably, the collection rates were relatively good when the angle was between 5° and 10° (i.e., 5°, 7°, and 10°), even more preferably, between 5° and 7°.

Note that, when the vent was present, the peak of angles appeared at 6.7° and 7° at which the collection rate was high, but such a peak was not observed when the vent was absent.

**[Table 7]**

| Vent | Present | Absent |
|---|---|---|
| Plasma collection rate | 30% | 13.5% |

**[Table 8]**

| End thickness cutting angle | 5° | 7° | 10° | 20° | 60° | 80° |
|---|---|---|---|---|---|---|
| Plasma collection rate (vent absent) | 13.9% | 13.5% | 13% | 4.5% | 0% | 0% |
| Plasma collection rate (vent present) | 12% | 30% | 12% | 6% | 0% | % |

### <Experiment 10> (Consideration of end shape in a width direction of blood separation member

Object: To consider the plasma collection rate for 100 µL of blood when the end of the blood separation member has a shape in the width direction tapering toward the tip end in a shape of a triangle (hereinafter referred to as "width shape of the end in a shape of a triangle" or simply "width shape of the end").

Method: A plasma collection rate of the blood for 100 µL of blood using separation member having a dimensions of 16 mm (length) × 8 mm (width) is examined, wherein the end of the separation member is cut so that it is narrowed toward the tip end (in a shape of triangle) (when seen from above, the end of the blood separation member is formed in a shape of a triangle with a tip end angle of 60°; see Fig. 6A)

Results: As indicated in Table 9, (1) When a cutting was carried out so as to form a width shape of the end in a shape of triangle (hereafter referred to "end width cutting in a shape of triangle" or simply " end width cutting" as well), but end width cutting for reducing the thickness was not carried out (Figs. 6A, 6B), the correction rate was 0%; (2) When the end width cutting was carried out and end thickness cutting was also carried out at 7° (Figs. 6A, 6C), the plasma collection rate was 6.5%.

In contrast, (3) When neither end width cutting nor end thickness cutting was carried out, the correction rate was 0%; (4) When the end thickness cutting was carried out without carrying out the end width cutting, the plasma collection rate was 30% which exhibited a remarkable efficiency, as was the case in experiment 3 (see Table 8; similar to Table 1 indicating the results of experiment 3).

In other word, as for the cutting for shaping the end, it was reconfirmed that the end thickness cutting at 7°of the blood separation member alone exhibited a remarkable efficiency in the plasma collection rate, in contrast, a result came out that the end width cutting had no effect on the plasma collection rate (0%). Moreover, the result showed that, when the end thickness cutting and the end width cutting were combined (i.e., above (2)), the remarkable efficiency of the end thickness cutting was inhibited, resulting in a sharp dropping in the plasma collection rate to 6.5%.

**[Table 9]**

| Amount of whole blood (µL) | End width cutting | End thickness cutting | Number of filters | Collection rate (%) |
|---|---|---|---|---|
| 100 | 60° vertex | No cut | 1 | 0% |
| 100 | 60° vertex | Cut (7°) | 1 | 6.5% |
| 100 | No cut | No cut | 1 | 0% |
| 100 | No cut | Cut (7°) | 1 | 30.0% |

Therefore, from the results of the experiment of the present embodiments, it is understood that, as for the cutting for shaping the end of a blood separation member, such end width cutting for narrowing the width of the blood separation member in a shape of a triangle has no effect, whereas the end thickness cutting for reducing the "thickness" of said member in a shape of a triangle is an important factor for remarkably improving the plasma collection effect.

### <Experiment 11> (Biochemical examination results of collected plasma)

Object: To consider biochemical nature of the plasma collected in embodiment 1 of the invention.

Method: a general biochemical examination of the blood plasma collected through methods using an automated biochemical analyzer (Hitachi 7180 model; Hitachi High-Technologies Communication/corporation) is conduct, one of the methods is the method of collecting the plasma using the embodiment subjected to the end thickness cutting, the other is a comparative example of method using centrifugal separation for collecting plasma (conventional example).

Results: The biochemical examination conducted on eight items of HDL, LDL, T-CHO, TG, ALP, T-BIL, CRE, and UA. As indicated in Table 10, it was understood that the values in the embodiment 1 were substantially approached to those in the comparative embodiment using the centrifugal separation method, and there were no problem in the biochemical nature of the plasma collected through the embodiment of the invention, thus an excellent results were obtained.

From above, it is shown that the plasma separation apparatus of the embodiment of the invention is effective as a sampling apparatus for the biochemical examination of blood.

**[Table 10]**

| | Examination items | HDL | LDL | T-CHO | TG | ALP | T-BIL | CRE | UA |
|---|---|---|---|---|---|---|---|---|---|
| | Units | mg/dL | mg/dL | mg/dL | mg/dL | U/L | mg/dL | mg/dL | mg/dL |
| Stand ards | Lower limit | 30 | 60 | 128 | 42 | 130 | 0.2 | 0.47 | 2.9 |
| | Upper limit | 85 | 119 | 250 | 168 | 350 | 1.2 | 1.09 | 7.7 |
| Blood A | Centrifuge **separation** | 50 | 101 | 171 | 256 | 309 | 1.28 | 0.74 | 4.88 |
| | Embodiment 1 | 49 | 98 | 168 | 275 | 313 | 1.29 | 0.80 | 4.91 |
| | % to centrifuge | 98.3% | 97.4% | 98.6% | 107.7% | 101.2% | 101.0% | 108.9% | 100.5% |
| Blood B | Centrifuge separation | 53 | 86 | 146 | 99 | 132 | 0.44 | 0.69 | 4.43 |
| | Embodiment 1 | 53 | 86 | 145 | 103 | 141 | 0.44 | 0.72 | 4.50 |
| | % to centrifuge | 99.4% | 99.5% | 99.4% | 104.2% | 106.2% | 100.0% | 104.1% | 101.6% |

Here, the dimensions for one mode of the embodiment are shown in Table 11.

**[Table 11]**

| | |
|---|---|
| External dimensions (mm) | 80 (W) × 25 (D) × 6 (H) |
| External material | ABS |
| Separating part pedestal dimensions (mm) | 20 (W) × 12 (D) × 1 (H) |
| Separating part pedestal material | ABS |
| Blood separation part dimensions (mm) | 16 (W) × 8 (D) × 0.4 (H) |
| Blood separation member material | Glass fiber |
| Collection part passage dimensions (mm) | 8 (W) × 55 (D) × 0.15 (H) |
| Collection part passage material | Glass |

### <Experiment 12> (Consideration of material of the plasma collection part and shape of the end of blood separation member)

Object: To consider a result of the plasma collection rates and the effectiveness of the end cutting of a blood separation member, when material of the plasma collection member is changed.

Method: the plasma collection rate by using a blood separation member made of polycarbonate (to which hydrophilic resin sputtering or plasma hydrophilizing treatment were applied) and subjected to the end thickness cutting at 7°, are examined, and further the plasma collection rate are examined when the end of the blood separation member was perpendicularly notched. Note that ten notches were provided.

Results: As indicated in Table 12, when the member of the collection part was made of hydrophilic-treated polycarbonate, the collection rate did not extend to one of which the collection part was made of glass (30%; see Table 1), yet a collection rate about 20% could still be obtained.

The blood separation member provided with ten notches was subjected to two experiments. Surprisingly, the collection rates were increased and their rates of increase were 1.3-times or 1.5-times, respectively. Especially in the experiment 13-1, the collection rate is 28.7% which was excellent.

From above, in embodiment of the invention, it is shown again that various resin materials can be adapted provided that a hydrophilization treatment is applied to the collection part.

Furthermore, it is understood that the collection rate is improved by further notching at the end in addition to the end thickness cutting at 7°, in the end working of the blood separation member.

**[Table 12]**

| | No notch | Ten notches |
|---|---|---|
| Experiment 13-1: collection rate (%) | 19.8 | 28.7 |
| Experiment 13-2: collection rate (%) | 17.1 | 21.7 |

### <Experiment 13> (Consideration of presence or absence of hemolysis)

Object: To consider presence or absence of hemolysis in a collection part.

Method: Presence or absence of hemolysis with respect to the plasma collected in the collection part in each of experiments was visually observed (except Experiments 5 to 13) .

Results: Hemolysis was not observed with respect to the collected plasma in each of the experiments. Note that, on rare occasion, however, hemolysis could be caused at the time when the blood was collected, but such case has been excluded from the present consideration.

### (Embodiment 2)

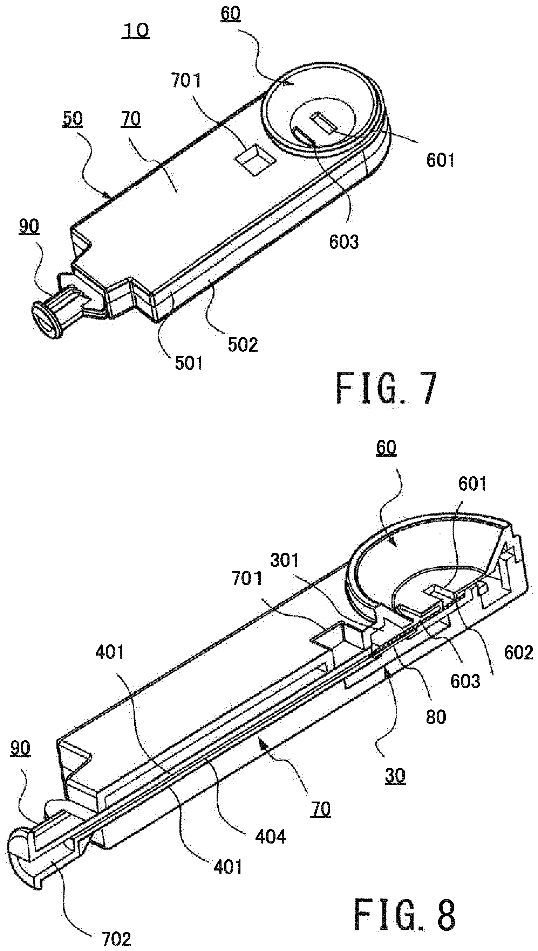

One of other embodiment of the invention is an improved type of the embodiment 1 and is a plasma separation apparatus 10 comprises at least a core part 20 and a housing part 50 (Fig. 7 to Fig.10).

The basic structure is the same as that of the embodiment 1, therefore, an identical name in each part indicates the same technical meaning as that described regarding to the above-mentioned embodiment 1.

### <External views and sectional views>

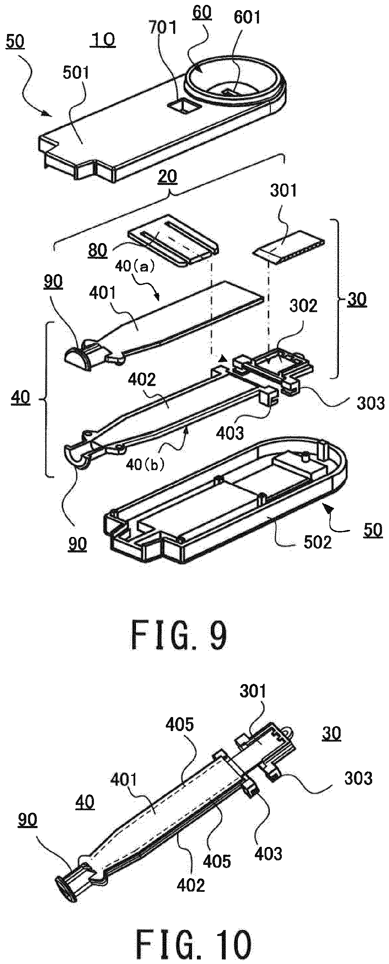

Fig. 7 shows an external view of the plasma separation apparatus 10 of the invention; Fig. 8 shows a sectional view; Fig. 9 shows an explosive view; and Fig. 10 shows a perspective view of the outer of a core part 30 after it is assembled (a support member 801 for separation not shown).

### <Overall external view> (see Fig. 7 to Fig. 10)

The housing part 50 containing the core part 20 is provided with at least a blood inlet part 60 and a collecting body part 70.

The blood inlet part 60 is provided at an upper part of a blood separation part 30 contained in the housing, and is provided with a blood inlet 601, a blood reserving part 602, and optionally, a blood deployment observation window 603 and other parts as needed.

The blood reserving part 602 has the effect of temporarily forming a blood reservoir, as mentioned above. With this, after blood is dropped, a part of it is prevented from slipping over the upper surface of the blood separation member, leaking therefrom (in this state, the blood cannot be separated by the blood separation member) and flowing into the plasma collection part (the blood is mixed into the plasma) . (Note that, the blood reserving part also has a function to exhibit the blood separating effectiveness of the blood separation member more effectively.)

In other words, a part of the dropped blood usually flows while forming the passage by the capillary phenomenon in the blood separation member, but the remaining blood would flood over the surface of the separation member which phenomenon can be caused not a few, and as a result, a part of the blood would flow into the collection part without separating. Therefore, to address this problem, such blood reserving part is disposed, so that the somewhat flooding blood on the member surface is temporarily reserved as is by the surface tension, and thereafter gradually flows into the separation member, so that a leaking phenomenon as mentioned above can be prevented.

Although the blood reserving part 602 is placed to be in contact with the upper surface of the blood separation member 301, a close contact with the blood separation member 301 is not necessary. Some gaps are allowed to be unless the blood reserving part loses its effectiveness as the blood reserving part (temporal blood reservoir) by surface tension and, unless the blood slips over the upper surface of the blood separation member, leaks therefrom, and flows into the collection part through the gaps.

Such the blood reserving part can be of any shape, can be made of any material, and can be placed anywhere, when its effect as the above-mentioned "temporal blood reservoir forming part" (or "guiding part for forming temporal blood reservoir") is maintained.

In the embodiment, the blood reserving part 602 is formed in a convex part extending along the shape of the blood inlet 601 and perpendicularly extending downward therefrom, but it may be, for example, circular, comb-shape, U-shape, or channel-shape, and may be bar-shaped like 33 shown in Fig. 4D. Furthermore, like the previous embodiment, it may be provided on the lower surface of the blood inlet 601 by being integral molded with the housing 50, or, for example, the bar-shaped like a member mentioned above may be integrally molded with a pedestal for a separating part 302 in a bridge shape. Note that the blood reserving part also has a function to exhibit the effectiveness of blood separation of the blood separation member to the maximum.

On the other hand, a collecting body part 70, it is provided with a plasma deployment observation window 701, an opening 702 or the like, as needed. The opening 702 may include a film that allows air to pass there-through but not liquid, a valve, a connector to an examination system or the like, or a liquid (plasma) reserving member. Here, as for ventilation, when any alternative is provided, the opening 702 is not necessarily required.

### <Structure of core part>

The structure of a core part 20 of the plasma separation apparatus 10 of the invention generally includes a blood separation part 30 and a plasma collection part 40 (Fig. 9).

The blood separation part 30 includes at least, a blood separation member 301, a pedestal 302 for blood separation part, and a receptor 303. The blood separation member 301 is mounted on the pedestal 302 for blood separation part (Fig. 9) and an end thereof is inserted into a gap between two plasma collection members (Fig. 3, Fig. 8).

The plasma collection part 40 is provided with at least two sheets of the plasma collection members 401, 402, both ends of those plasma collection members 401, 402 are sealed by such means as an adhesive or the like, so that a plasma collecting area 404 surrounded by the plasma collection members 401, 402 and the adhesive part is formed (Fig. 8 to Fig. 10).

As adhesive member, an adhesive tape, an adhesive, and the like may be considered. Integral molding without using the adhesive may be carried out, when the adhesive member is made of the same material as that of the correcting member.

When a blood specimen is dropped through the blood inlet 601 of the blood inlet part 60 onto the blood separation part 30, the blood gradually infiltrates into the blood separation member 301, but it does not immediately infiltrate into the blood separation member 301, a flooded part of blood specimen over the upper surface of the blood separation member is temporarily reserved in the blood reserving part 602, while it gradually infiltrates into and spreads through the blood separation member 301 due to the wicking effect caused by a complex fiber structure of the blood separation member 301, which eventually generates a force to push it toward the plasma collection part 40 connected with the end of the blood separation member 301, so that a collection part passage is formed from the blood separation part 30 to the plasma collecting area 404 through the corporation of the force and a capillary phenomenon of the gap (between the plasma collection members 401) of the plasma collection part 40.

Cellular components of blood (red blood cells and white blood cells and the like) are trapped in the blood separation member 301, on the other hand, a liquid component of the blood (plasma or serum and the like) flows into the plasma collecting area 404 in the plasma collection part 40, while forming a collection part passage through the communication/corporation between the wicking effect in the blood separation member 301 and the capillary phenomenon of the gap of the plasma collection part 40, thereby clearly separated from the cellular components and collected. (Although the reference signs in the drawings are affixed for assisting in understanding, the present embodiment aspect is not limited by these reference signs.)

Note that the plasma collection part 40 may be provided with an accessory part 90 at a distal end. One mode of the accessory part 90 may be provided with a connecting part (connector) for connecting to the examination system, an outer container or the like, the liquid (plasma) reservoir, valve or the like.

### <Separation of plasma collection part by operating the support for separating part>

As is shown in Fig. 9, the receptors 303 and 403 respectively receive members of a supporting part 80 for separation to interconnect the pedestal 302 for blood separation and the plasma collection member 402, and integrate core part 20 (the blood separation part 30 and the plasma collection part 40). After the completion of plasma separating, the supporting part 80 for separation is removed to disassemble the core part 20, so that the plasma collection part 40 can be separated.

The structure of the supporting part 80 for separation can be any form when the plasma collection part and the blood separation part can be safely separated. For example, without providing the support member for separation, the core part may be configured that the plasma collection part and the blood separation part can be cut off.

Materials for the blood separation member 301 are not limited to those in the above-mentioned embodiment 1, for example, fiber composed of glass or synthetic polymers having a small fiber diameter, laminated porous polymeric sheet or thin nonwoven fabric and the like can be used when they have a function and effectiveness as a blood separation member.

On the other hand, As for materials of the pedestal 302 for separating part and the plasma collection members 401, 402, the same materials as in embodiment 1 may be used, but from viewpoints of cost and manufacture, it is preferable to design to make it by using a material such as resin and the like which may be easily molded.

Nevertheless, in order to fully realize the blood separation effect of the present embodiment, it should be also taken into consideration to impart hydrophobicity to a surface of the pedestal for separating part to prevent blood from adhering and impart hydrophilicity to a surface of the plasma collection part with which plasma would come into contact to exhibit an optimal effectiveness of the capillary phenomenon, as in embodiment 1.

### <Method for separating plasma from blood without requiring centrifuge separator, suctioning or pressurizing apparatus, or the like-2> See Fig. 3.

A method for separating plasma from blood using a plasma separation apparatus of the invention comprises at least the following steps:
1. A step for providing a plasma separation apparatus, wherein the end of the blood separation member is inserted into (arranged in) a gap of the plasma collection part, the blood separation member being included in the blood separation part and mounted on a hydrophobic pedestal, and a cross-sectional area of the end being gradually decreased toward a plasma collection part (preparation of a blood separation apparatus) ;
2. A step for introducing blood to the blood separation member through a blood reserving part serving as a temporal blood reservoir (a step for applying blood);
3. A step for separating blood introduced in the blood separation member, wherein the blood is separated into blood cellular components and plasma while forming a passage in the blood separation member owing to a wicking effect in the blood separation member (a step for blood deployment and separation) ;
4. A step for collecting plasma in the plasma collection part, wherein the separated plasma forms a passage in the gap of the plasma collection part through the communication/corporation between the wicking effect in the blood separation member and a capillary phenomenon in the plasma collection part, so that the plasma is collected in the plasma collection part (a step for collecting plasma).
5. A step for disassembling a housing, extracting a core part contained therein, and separating the plasma collection part and the blood separation member by means of a support member for separation.
   The following step is the last for conducting a final examination.
6. A step for sending the plasma collection part (and/or the blood separation member) to the examination facilities for examination.

Because this method does not require any special apparatus such as a centrifuge separator, a suctioning or pressurizing apparatus (tool), or the like and can be performed by an ordinary parson without training who is not a medical worker, it can deal with needs of a person at home, a resident in a remote place or in developing countries, or a minority or a majority of people, plasma separation can be performed easily and quickly and separated plasma can be send to the examination facilities as it is. Thus, the apparatus is very useful.

Note that, although a plasma separation apparatus of the invention has been particularly described with reference to the separation of plasma, it can be also used for an examination of blood cells trapped in a blood separation member, in particular, an examination of HbA1c and the like using red blood cells, an examination of presence/absence of HIV-B using white blood cells and an examination regarding to immune cells and the like.

A plasma separation apparatus of the invention may be formed as a blood examination kit, together with a minutia amount blood collecting device (blood sampling device).

One embodiment of the invention is a separation apparatus for blood. It is a plasma separation apparatus when the blood sampling device includes an anticoagulant, or it is a serum separation apparatus when the blood sampling device does not include an anticoagulant.

A plasma separation apparatus of the invention does not require a special apparatus such as a centrifuge separator, a suctioning or pressurizing apparatus (tool), or the like, and is easy to handle even the minute amount of blood and available for an examination for medical care at home or in any remoted area or in developing countries at a low cost.

Ultimately, one can pack the separated plasma collection part and/or blood separation part in a proper manner and send to examination facilities so that it is subjected to blood examination.

Preferable embodiments of the invention have been described with reference to the drawings; however, the invention is not limited by such embodiments but can be modified without departing from the spirit of the inventive.

It is obvious that those skilled in the art will come to various modifications and variations within the spirit of the invention set forth in the attached claims; therefore, naturally, all those modifications and variations are deemed to be included in the technical scope of the invention.

A part of or all of the above-mentioned embodiments may be also described as the following supplementary notes, but without limitation.

(Supplementary note A) A plasma separation apparatus, comprising, a blood separation part having a blood separation member, and a plasma collection part having a plasma collection member, wherein the blood separation member is mounted on a hydrophobic pedestal and includes a blood receiving area and a plasma separation area that is connected to the plasma collection part, and the plasma separation part has the cross-sectional area thereof gradually decreasing toward the plasma collection part.

(Supplementary note B) The plasma separation apparatus according to Claim A, wherein the cross-sectional area of the plasma separation area is gradually decreased toward the plasma collection part by the end cutting.

(Supplementary note C) The plasma separation apparatus according to claim A or B, wherein the blood separation part comprises a blood reserving part on the upper surface side of the blood separation member for temporarily reserving blood.

(Supplementary note D) The plasma separation apparatus according to any one of claims A to C, wherein the blood separation part and the plasma collection part together constitute a core part, wherein the core part is contained in a housing part.

(Supplementary note E) The plasma separation apparatus according to any one of claims A to D, further comprising a support member for separation, for separating into the blood separation part and the plasma collection part.

(Supplementary note F) A blood examination kit, comprising the plasma separation apparatus according to any one of claims A to E and a blood collection tool.

(Supplementary note G) A plasma separation method for separating plasma using a plasma separation apparatus comprising a blood separation part having a blood separation member and a plasma collection part having a plasma collection member, wherein
the blood separation member arranged on a hydrophobic pedestal has a blood receiving area and a plasma separation area connected to the plasma collection part, a cross-sectional area of the plasma separation area is gradually decreased toward the plasma collection part;
the plasma separation method comprising steps of ;
separating the plasma from the blood received in the blood receiving area, in the plasma separation area, and
collecting the separated plasma in the plasma collection part.

(Supplementary note H) The plasma separation method according to claim G, wherein the cross-sectional area of the plasma separation part is gradually decreased toward the plasma collection part by the end cutting.

(Supplementary note I) The plasma separation method according to claim G or H, wherein the blood separation part comprises a blood reserving part on the upper-surface side of the blood separation member so that blood is received in the blood receiving area after temporarily being reserved in the blood reserving part.

(Supplementary note J) The plasma separation method according to any one of claims G to H, comprising a step of; separating the blood separation part and the plasma collection part after the plasma separated from the blood in the plasma separation area is collected in the plasma collection part.

(Supplementary note a) A plasma separation apparatus comprising a blood separation part and a plasma collection part, wherein the blood separation part has a blood separation member, a blood reserving part for temporarily reserving blood on the upper surface of the blood separation member, and a hydrophobic pedestal for supporting the blood separation member, the plasma collection part is formed with a plasma collecting area which is a gap between collecting members; and an end of the blood separation member on the plasma collection part side is inserted into the gap in the plasma collection part, a cross-sectional area of the end being gradually decreased toward the plasma collection part.

(Supplementary note b) The plasma separation apparatus according to claim a, wherein, owing to the insertion of the end of the blood separation member on the plasma collection part side into the gap in the plasma collection part, the plasma can be collected in the plasma collection part through the communication/corporation between a wicking effect in the blood separation member and a capillary phenomenon of the gap of the plasma collection part, when blood is dropped onto the blood separation member.

(Supplementary note c) The plasma separation apparatus according to claim a or b, further comprising a support member for separation, for separating the blood separation part and the plasma collection part.

(Supplementary note d) A plasma separation method comprising steps of: providing a plasma separation apparatus in which an end of a blood separation member on a plasma collection part side is inserted into a gap in the plasma collection part;
introducing blood to the blood separation member through a blood reserving part serving as a temporal blood reservoir from a blood inlet of a blood inlet part;
separating the blood introduced into the blood separation part into cellular and plasma components, as the blood is developed while forming a passage in the blood separation part owing to an wicking effect of the blood separation member; and
collecting the separated plasma component in the plasma collection part as a liquid component, the separated plasma component forming the passage in the gap in the plasma collection part through the communication/corporation between the wicking effect of the blood separation member and a capillary phenomenon of the plasma collection part.

(Supplementary note e) The plasma separation method according to claim d comprising; a step of separating the plasma separation apparatus into the blood separation part and the plasma collection part after the completion of plasma collection.

### Industrial Applicability

The invention is useful in separating blood components to prepare a sample for blood examination easily, conveniently, and safely at a low cost even in the absence of a medical worker.

### Reference Signs List

1 plasma separation apparatus
2 core part
3 blood separation part
   31 blood separation member
   32 the pedestal for separating part (hydrophobic surface)
   33 blood reserving part (blood reservoir)
4 plasma collection part
   41a plasma collection member
   41b plasma collection member
   42 edge
   43 adhesive member
   44 plasma collecting area
   45 opening (vent)
   46 collection part passage
5 housing
6 blood inlet part
   61 blood inlet
   62 blood reserving part (blood reservoir forming (guiding) part)
   63 blood deployment observation window
7 collecting body part
   71 plasma deployment observation window
   72 opening
10 plasma separation apparatus
20 core part
30 blood separation part
   301 blood separation member
   302 the pedestal for separating part (hydrophobic surface)
   303 receptor a
40 plasma collection part
   40a collection part cover
   40b collection part body
   401 plasma collection member
   402 edge
   403 receptor b
   404 plasma collecting area
50 housing
   501 housing cover
   502 housing base
60 blood inlet part
   601 blood inlet
   602 blood reserving part (blood reservoir forming (guiding) part)
   603 blood deployment observation window
70 collecting body part
   701 plasma deployment observation window
   702 opening
80 supporting part for separation
90 accessory part

## Claims

1. A plasma separation apparatus, comprising, a blood separation part having a blood separation member, and a plasma collection part having a plasma collection member, wherein the blood separation member is mounted on a hydrophobic pedestal and includes a blood receiving area and a plasma separation area that is connected to the plasma collection part, and the plasma separation part has the cross-sectional area thereof gradually decreasing toward the plasma collection part.

2. The plasma separation apparatus according to claim 1, wherein the cross-sectional area of the plasma separation area is gradually decreased toward the plasma collection part by the end cutting.

3. The plasma separation apparatus according to claim 1 or 2, wherein the blood separation part comprises a blood reserving part on the upper surface side of the blood separation member for temporarily reserving blood.

4. The plasma separation apparatus according to any one of claims 1 to 3, wherein the blood separation part and the plasma collection part together constitute a core part, wherein the core part is contained in a housing part.

5. The plasma separation apparatus according to any one of claims 1 to 4, further comprising a support member for separation, for separating into the blood separation part and the plasma collection part.

6. A blood examination kit, comprising the plasma separation apparatus according to any one of claims 1 to 5 and a blood collection tool.

7. A plasma separation method for separating plasma using a plasma separation apparatus comprising a blood separation part having a blood separation member and a plasma collection part having a plasma collection member, wherein
the blood separation member arranged on a hydrophobic pedestal has a blood receiving area and a plasma separation area connected to the plasma collection part, a cross-sectional area of the plasma separation area is gradually decreased toward the plasma collection part;
the plasma separation method comprising steps of ;
separating the plasma from the blood received in the blood receiving area, in the plasma separation area, and
collecting the separated plasma in the plasma collection part.

8. The plasma separation method according to claim 7, wherein the cross-sectional area of the plasma separation part is gradually decreased toward the plasma collection part by the end cutting.

9. The plasma separation method according to claim 6 or 7, wherein the blood separation part comprises a blood reserving part on the upper-surface side of the blood separation member so that blood is received in the blood receiving area after temporarily being reserved in the blood reserving part.

10. The plasma separation method according to any one of claims 7 to 9, comprising a step of;
separating the blood separation part and the plasma collection part after the plasma separated from the blood in the plasma separation area is collected in the plasma collection part.
